(19) Europäisches Patentamt
European Patent Office
Office européen des brevets

(11) Publication number: 0 122 042 B1

(12) **EUROPEAN PATENT SPECIFICATION**

(45) Date of publication of patent specification: **02.11.89**

(51) Int. Cl.[4]: **A 61 F 13/00,** A 61 F 13/18, A 41 B 13/02

(21) Application number: **84301578.5**

(22) Date of filing: **09.03.84**

(54) **High density absorbent structures, method of their manufacture and absorbent products containing them.**

(30) Priority: **10.03.83 US 473846**
**24.06.83 US 507824**
**06.09.83 US 529900**

(43) Date of publication of application:
**17.10.84 Bulletin 84/42**

(45) Publication of the grant of the patent:
**02.11.89 Bulletin 89/44**

(84) Designated Contracting States:
**AT BE CH DE FR IT LI LU NL SE**

(56) References cited:
**FR-A-2 293 913**
**GB-A- 549 213**
**GB-A-1 192 581**
**GB-A-1 548 156**
**US-A-3 381 688**
**US-A-3 971 379**
**US-A-4 102 340**

(73) Proprietor: **THE PROCTER & GAMBLE COMPANY**
**301 East Sixth Street**
**Cincinnati Ohio 45201 (US)**

(72) Inventor: **Weisman, Paul Thomas**
**5861 Lake Tahoe Court**
**Fairfield Ohio 45014 (US)**
Inventor: **Goldman, Stephen Allen**
**1579 Acreview Drive**
**Cincinnati Ohio 45240 (US)**

(74) Representative: **Gibson, Tony Nicholas et al**
**Procter & Gamble (NTC) Limited Whitley Road**
**Longbenton Newcastle upon Tyne NE12 9TS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 122 042 B1

## Description

This invention relates to flexible, substantially unbonded, absorbent structures comprising a mixture of hydrophilic fibers and discrete particles of a water-insoluble hydrogel. Flexible absorbent structures, generally non-woven sheets or fibrous webs, have the ability to absorb significant quantities of fluids like water and body exudates. They are used, for example, as disposable towels, facial tissues, toilet tissues, or as adsorbent cores in absorbent products like disposable diapers and sanitary napkins. Generally, such structures are made of inexpensive hydrophilic fibers, typically wood pulp fibers.

Water-insoluble hydrogels are polymeric materials which are capable of absorbing large quantities of water, typically more than 20 times their own weight. When first introduced, these materials were expected to generate a major breakthrough in the world of disposable absorbent consumer products (i.e. products like disposable diapers, sanitary napkins, incontinent pads, and the like). Yet, up to this day, no large-scale use of water-insoluble hydrogels in disposable absorbent products has taken place. The reason is that, in spite of the extremely high water absorption of hydrogels, their performance when used in disposable absorbent products has been unacceptable.

One cause of the poor performance of hydrogels is a phenomenon called gell blocking. The term gel blocking describes a phenomenon that occurs when a hydrogel particle, film, fiber, etc. is wetted; the surface swells and inhibits liquid transmission to the interior. Wetting of the interior subsequently takes place via a very slow diffusion process. In practical terms this means that the absorption is much slower than discharge of fluid to be absorbed, and failure of a diaper or sanitary napkin or other absorbent structure may take place well before the hydrogel material in the absorbent structure is fully wet.

Water-insoluble hydrogels have a water absorbent capacity which far exceeds, generally by far more than an order of magnitude, the absorbent capacity for water of wood pulp fibrous webs which are typically used in disposable absorbent consumer products. The absorption capacity for an eletrolyte containing fluid, like urine, is much less but still up to about an order of magnitude higher than that of fibrous webs. Many workers in the field have therefore attempted to somehow incorporate hydrogel materials into wood pulp fiber webs in order to increase the fluid absorption capacities of such webs. Early attempts involved simple mixing of hydrogel powder into the fibrous web. This approach did not lead to any increase of the bulk absorption capacity of the web. (See, for example, R. E. Ericson, "First International Absorbent Products Conference Proceedings", November, 1980, Section 6 at page 3). Ericson reports that "fluid retention under pressure is increased but bulk capacity remains essentially the same". Several explanations for this phenomenon have been given. Ericson ascribes it to the fact that the fibrous matrix prevents swelling of the hydrogel particles. Others believe that the very poor wicking characteristics of hydrogels are responsible for the disappointing performance. Whatever the cause may be, i⁺ is well established that simple mixtures of hydrophilic fibers and hydrogel particles do not have the absorption capacity one would expect on the basis of the respective contributions of the components of such mixtures.

Based ùpon the assumption that the poor wicking of hydrogels causes their poor performance in disposable absorbent structures, some workers in the field have attempted to improve hydrogel performance by introducing fibers into the hydrogel particles. This may be achieved by wet laying of mixtures of hydrogel particles and hydrophilic fibers. During the wet stage of such a process the hydrogel swells. During the drying step the hydrogel tends to retract. As a result the gel spreads over the fiber surface and creates fiber-fiber bonds, in a manner not dissimilar from the bonding which occurs when binders (e.g. latex) are used. As a result of the wet treatment and the bonding by the hydrogel, the resulting absorbent structure is very stiff. It has been disclosed that the stiffness of such structures may be reduced by subjecting the structure to a high pressure. Even when so treated the stiffness of such structures is still relatively high, especially when fiber/hydrogel ratios of more than 50:50 are used. Such fiber/hydrogel ratios are, however, very desirable from a cost standpoint: hydrogel is far more expensive than, for example, wood pulp fibers. Moreover, the art-disclosed processes involve the handling of large amounts of water and subsequent drying. This adds significantly to the manufacturing costs of the absorbent structures.

An alternative form of absorbent structure is that in which the hydrogel material is dispersed in the dry-state in the fibrous absorbent pad. A laminated structure of this type is described by Mesek et al US—A—4102340 which discloses an absorbent article such as a diaper or sanitary napkin comprising a facing sheet, a moisture impervious backing sheet and a multi-layer absorbent pad means disposed therebetween. The absorbent pad means comprises a fibrous structure having an intermediate densified layer and a layer of highly porous loosely compacted batt on both sides of the densified layer. The batt layer between the densified fibrous layer and the backing sheet contains a particulate water-insoluble, water-swellable polymeric absorbent. The densified layer is formed by calendering an airlaid fibrous batt, exposing one surface to a fine spray of moisture and then compressing the layer by a further calendering operation. The overall density of the fibrous stucture is from 0.1 to 0.15 g/cc.

Another approach has been to form laminated structures, whereby a layer of hydrogel material is placed against a layer of a material having good wicking properties. The wicking layer spreads the liquid over a larger surface of the hydrogel layer, so that more of the hydrogel is exposed to the liquid to be absorbed. It has been claimed that such structures provide a higher absorption capacity than e.g. mixtures of hydrogel particles in hydrophilic fibrous webs. The wicking layer provides spreading of the liquid across

the surface of the hydrogel layer, but does not ensure penetration into the hydrogel layer. The latter liquid movement is still severely limited by gel blocking. In other words, absorbent structures as they are known in the art fail to fully exploit the absorption potential of hydrogels.

The gel blocking phenomenon has been well documented, and the resulting poor properties of absorbent structures comprising hydrogels have been discussed: see, for example, E. Carus, "First International Absorbent Products Conference Proceedings", November, 1980, Section V-1; and J. H. Field, "Pulp Parameters Affecting Product Performance", TAPPI, *65*(7) *1982*, pp. 93—97.

Japanese Patent Specification 56—65630, published June 3, 1981, discloses a process for preparing "tufted lumps" of cellulose fiber holding water-insoluble resins. The lumps are prepared by dispersing the fibers and the resin in methanol, wet-laying the mixture and drying off the solvent. The web is subsequently compressed to a density of more than 0.1 g/cm$^3$, preferably about 0.6 g/cm$^3$. The sheet thus obtained is cut into pieces of less than 0.5 g each. A similar approach is taken by Kopolow, U.S. Patent 4,354,901, issued October 19, 1982. This reference discloses a process whereby a slurry is formed of less than about 0.1% by weight of solids in water, the solids being a mixture of cellulose fibers and particulate hydro-colloidal material. A wet web is formed from the slurry which is subsequently dried and densified by at least 10%, preferably at least 50%. It is said that the densifying step results in reduction of the stiffness of the absorbent structure (Gurley Stiffness values of less than 40 g).

There is therefore a continuing need for absorbent structures which are flexible and which more fully exploit the absorbent capacity of hydrogels than has heretofore been possible. The absorbent structures of the present invention provide superior absorbent capacity and excellent wicking properties, and yet are flexible, resilient, and have good lateral integrity. These structures are uniquely adapted for use in disposable diapers which are extremely thin and comfortable but which have an absorbent capacity which is at least equal to the much bulkier products which are currently marketed. The absorbent structures can be made by a process which does not involve water or another solvent. The process therefore does not involve the handling of solvents, or drying. The simplicity of the process permits the use of standard equipment as is currently being used for the manufacture of absorbent webs; it is possible to implement the manufacture of the absorbent structures of the present invention without any major capital investments, and at low per unit manufacturing costs. It is therefore an object of this invention to provide a flexible absorbent structure which comprises a water-insoluble hydrogel, having improved absorbent properties. It is a futher object to provide improved disposable absorbent products, such as diapers, which are substantially thinner and less bulky than conventional disposable absorbent products. It is a further object of this invention to provide a process for making such absorbent structures.

This invention relates to an absorbent structure comprising a mixture of hydrophilic fibers and water-insoluble hydrogel in the form of discrete particles of cross-linked polymeric material in a fiber hydrogel weight ratio of from 30:70 to 98:2, wherein the structure comprises a fiber-particle mixture which is air laid to form a flexible substantially unbonded structure with a moisture content of less than 10% by weight and a density of from 0.15 to 1 g/cm$_3$.

The basis of this invention is the discovery that mixtures of hydrophilic fibers and particles of water-insoluble hydrogels may be formed into flexible, highly absorbent structures, provided that the weight ratio of fiber/hydrogel is between 0:70 to 98:2, and further provided that the structure is densified to a density of from 0.15 to 1 g/cm$^3$. The absorbent structures of the present invention are basically webs of hydrophilic fibers, having dipersed therein discrete particles of the water-insoluble hydrogel. The hydrogel particles may be randomly dispersed, or in a pattern of areas with a low fiber/hydrogel ratio, and areas of a high fiber/hydrogel ratio (which includes area of fiber alone).

By "substantially unbonded" is meant that the number of fiber/fiber bonds, fiber/hydrogel particle bonds and hydrogel particle/hydrogel bonds is kept as low as reasonably possible. Bonds, which may occur include hydrogen bonds (like paper-making bonds), other types of chemical bonds as may occur between fibers and hydrogel particles, among hydrogel particles, and among certain types of fibers (e.g. thermoplastic fibers) and mechanical bonds. This is important because the high absorbent capacities of the absorbent structures of the present invention are due to a significant extent to their ability to quickly regain volume upon initial wetting. A large number of bonds among the constituents of the structure would seriously impair this ability.

It is virtually impossible to entirely prevent bonds from being formed. However, some modest degree of bonding does not appear to negatively affect the structures' ability to quickly regain volume upon initial wetting. Generally, the degree of bonding is minimized by avoiding exposure of the fibers and hydrogel particles, or the absorbent structures, to water in its liquid form, and by avoiding prolonged exposure to air which has a high relative humidity. These process parameters are discussed in more detail hereinbelow.

By "hydrogel" as used herein is meant an inorganic or organic compound capable of absorbing aqueous fluids and retaining them under moderate pressures. For good results, the hydrogels must be water insoluble. Examples are inorganic materials such as silica gels and organic compounds such as cross-linked polymers. Cross-linking may be by covalent, ionic, vander Waals, or hydrogen bonding. Examples of polymers include polyacrylamides, polyvinyl alcohol, ethylene maleic anhydride copolymers, polyvinyl ethers, hydroxypropyl cellulose, carboxymethyl cellulose, polyvinyl morpholinone, polymers and copolymers of vinyl sulfonic acid, polyacrylates, polyacrylamides, polyvinyl pyridine and the like. Other suitable hydrogels are those disclosed in U.S. Patent 3,901,236, issued to Assarsson et al., August 26, 1975.

Particularly preferred polymers for use herein are hydrolyzed acrylonitrile grafted starch, acrylic acid grafted starch, polyacrylates, and isobutylene maleic anhydride copolymers, or mixtures thereof.

Processing for preparing hydrogels are disclosed in U.S. Patent 4,076,663, issued February 28, 1978 to Fusoyoshi Masuda et al.; in U.S. Patent 4,286,082, issued August 25, 1981 to Tsuno Tsubakimoto et al.; and further in U.S. Patents 3,734,876, 3,661,815, 3,670,731, 3,664,343, 3,783,871 and Belgian Patent 785,858.

As used herein "Particles" include particles of any shape, e.g. spherical or semi-spherical, cubic, rod-like, polyhedral etc.; but also shapes having a large greatest dimension/smallest dimension ratio, like needles, flakes and fibers, are contemplated for use herein. By "particle size" as used herein is meant the weight average of the smallest dimension of the individual particles. Conglomerates of hydrogel particles may also be used, provided the weight average size of such conglomerates is within the limits set forth hereinbelow.

Although the absorbent structures of the present invention are expected to perform well with hydrogel particles having a particle size varying over a wide range, other considerations may preclude the use of very small or very large particles. For reasons of industrial hygiene, (weight) average particle sizes smaller than 30 micrometers are less desirable. Particles having a smallest dimension larger than 4 mm may cause a feeling of grittiness in the absorbent structure, which is undesirable from a consumer standpoint. Preferred for use herein are particles having an (weight) average particle size of from 50 micrometers to 1 mm.

The type of hydrophillic fibers is not critical for use in the present invention. Any type of hydrophilic fiber which is suitable for use in conventional absorbent products is also suitable for use in the absorbent structure of the present invention. Specific examples include cellulose fibers, rayon, polyester fibers. Other examples of suitable hydrophilic fibers are hydrophilized hydrophobic fibers, like surfactant-treated or silica-treated thermoplastic fibers. Also, fibers which do not provide webs of sufficient absorbent capacity to be useful in conventional absorbent structures, but which do provide good wicking properties, are suitable for use in the absorbent structures of the present invention. This is so because, for the purposes of the present invention, wicking properties of the fibers are far more important than their absorbent capacity. For reasons of availability and cost, cellulost fibers, in particular wood pulp fibers, are preferred.

The relative amount of hydrophilic fibers and hydrogel particles are most conveniently expressed in a weight ratio fiber/hydrogel. These ratios may range from 30:70 to 98:2. Low fiber/hydrogel ratios, i.e. from 30:70 to 50:50, are practicable only when the hydrogel used possesses a low swelling capacity i.e., hydrogels having an absorbent capacity for urine and other body fluids of less than 15 times their own weight (15×). (Absorbent capacity data are generally available from the manufacturer of the hydrogel, or may conveniently be determined by means of the absorption/desorption test described hereinbelow). Hydrogels which have a very high absorption capacity (i.e. 25×, and which consequently exhibit a high degree of swelling after wetting) tend to gel block when used in absorbent structures at low fiber/hydrogel ratios, which causes undesirable, slow, diffusion type absorption kinetics. Very high fiber/hydrogel ratios, e.g. above 95:5 on the other hand, provide meaningful performance benefits only if the hydrogel used has a high absorbent capacity (e.g., 25× for urine and other body fluids). For most commercially available hydrogels the optimum fiber/hydrogel ratio is in the range of from 50:50 to 95:5.

Based on a cost/performance analysis, fiber/hydrogel ratios of from 75:25 to 90:10 are preferred. This preference is, of course, based on the relative costs of hydrophilic fibers (e.g. wood pulp fibers) and hydrogel. If, for example, wood pulp prices would go up and/or hydrogel prices would come down, lower fiber/hydrogel ratios would be more cost effective.

The density of the absorbent structure is of critical importance. When hydrogel particles are dispersed into an absorbent web of hydrophilic fibers having a density of 0.1 g/cm$^3$, the admixture of the hydrogel results in only a small increase in the amount of fluid which is absorbed within a practicably reasonable time (e.g. 10 minutes) because the fluid uptake of such webs is slow. When the absorbent structure is densified to a density of at least 0.15 g/cm$^3$, a marked increase in absorbent capacity is observed. Moreover, the fluid uptake becomes much faster upon densification. The capacity increase is surprising because densifying the web will result in reducing the void volume of the dry structure. It is believed that densifying the web results in better wicking of fluid into the web, so that more hydrogel particles participate in the absorption process, which results in a higher actual absorbent capacity. It is further believed that a densified web may be more effective in keeping the hydrogel particles isolated from each other. Densifying the web further, from 0.15 g/cm$^3$ to 1 g/cm$^3$, results in a reduction in the bulk of the structure (which is desirable from a consumer standpoint, for aesthetics reasons), without loss of absorbent capacity. However, above a density of 0.6 g/cm$^3$, further densification hardly reduces the bulk further, because of the inverse relationship between bulk and density. The densities of the absorbent structures of the present invention are therefore preferably in the range of from 0.15 to 0.6 g/cm$^3$, and more preferably within the range of from 0.25 to 0.4 g/cm$^3$.

The continuous flexible absorbent structures of the present invention can be made by a process comprising the steps of (a) air-laying a dry mixture of hydrophilic fibers and particles of a water-insoluble hydrogel in a weight ratio of from 30:70 to 98:2; and (b) compressing the web to a density of from 0.15 to 1 g/cm$^3$. Step (a) may be accomplished by metering an air flow containing hydrophilic fibers and an air flow containing hydrogel particles onto a wire screen. The fibers and the particles become mixed by turbulence of the two air flows as they meet. Alternatively, the fibers and the hydrogel may be mixed in a separate mixing chamber prior to air-laying.

For the purpose of the present invention is it essential that dry hydrogel particles are used. Also, neither the fibers, the particles nor the mixture of fibers and particles should be exposed to water in its liquid form, or another solvent, at any time during this process or subsequent thereto. When wet hydrogel particles are used, the fibers tend to become entangled and/or bonded with the particles which results in undesirable stiffness of the absorbent structure. Especially when cellulose fibers, e.g. wood pulp fibers, are used as the hydrophilic fibers in the absorbent structures of the present invention, the softness of these structures can be improved by adding small quantities of chemical debonding agents (cationic, nonionic or anionic surfactants) to the fibers. Examples of suitable debonding agents are disclosed in U.S. Patent 3,821,068, issued in June 28, 1974 to Shaw.

Particularly suitable debonding agents are quaternary ammonium compounds of the type disclosed in U.S. Patent 3,554,862, issued January 12, 1971 to Hervey et al.

Preferred quarternary ammonium compounds are those having the general formula

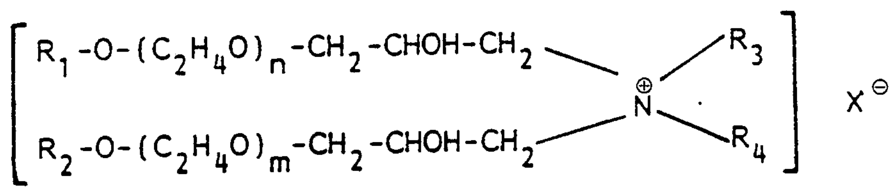

wherein $R_1$ and $R_2$ are hydrocarbyl groups containing from 8 to 22 carbon atoms, $R_3$ and $R_4$ are alkyl having from 1 to 6 carbon atoms; n and m are integers from 2 to 10, and X is halogen. Examples of such compounds are disclosed in U.S. Patent 4,144,122, issued March 13, 1979 to Emanuelsson et al.

Typically, the amount of chemical debonding agent in the absorbent structures is from .01% to 0.5% by weight of the hydrophilic fibers.

As used herein, "dry" does not mean "absolutely water-free". For example, under normal storage and handling conditions, hydrogel particles take up some moisture. The hydrophilic fibers also take up some moisture during storage. Furthermore, it may be desirable to use humidified air for air transport of the fibers and the hydrogel particles, to avoid dusting. Under such process conditions, the hydrogel particles and the fibers will take up even more moisture, but this does not negatively affect the practice of the present situation. However, contact times of the hydrogel with conveying air are short, and the limited water-uptake by the hydrogel during air-conveying with humidified air will not result in substantial bonding of the structure. The important criterion is that the hydrogel particles should not be allowed to swell appreciably, and should not develop a surface stickiness to an extent that it results in entanglement and/or bonding of the fibers. Generally, this can be achieved by exposing the hydrophilic fibers and the hydrogel particles only to water vapor, and not to water in its liquid form. Even mere exposure of the hydrogel to humidified air may result in substantial bonding of the structure during subsequent processing, especially during calendering, if such exposure is prolonged. For example, in U.S. Patent 4,252,761, issued February 24, 1981 to Schoggen et al., the entire thrust is to expose specific hydrogel materials to levels of water which result in bonded structures which are unacceptable for the purpose of the present invention due to unacceptable initial absorption kinetics. In order to ensure that the structure remains substantially unbonded the moisture content of the absorbent structure must be less than 10% by weight of the dry absorbent structure.

The absorbent structures may conveniently be made by using conventional equipment designed for air laying of hydrophilic fibrous webs. In such equipment, webs are typically formed by taking up hydrophilic fibers in air flow and depositing the fibers on a wire mesh screen. By metering the desired quantities of hydrogel particles into the air flow at a point just upstream of the wire mesh screen, the desired mixture of hydrophilic fibers and hydrogel particles can be made. The web formed on the screen is then passed through calender rolls which are set to a nip pressure resulting in the desired density of the absorbent structure. It will be clear that this embodiment of the process requires only minor modifications of conventional equipment for the manufacture of absorbent structures, i.e. installing a metering device for the addition of the hydrogel particles. In certain instances it may be necessary to replace the standard wire mesh screen on the equipment with one of a finer mesh size. This need will arise when relatively small hydrogel particles are used, and/or when the mesh size of the standard screen is relatively coarse.

Because of their particular properties, the absorbent structures of this invention are extremely suitable for use in disposable absorbent products. By "absorbent product" herein is meant a consumer product which is capable of absorbing significant quantities of water and other fluids, like body fluids. Examples of absorbent products include disposable diapers, sanitary napkins, incontinence pads, paper towels, facial tissues, and the like. As compared to conventional hydrophilic fibrous webs, the absorbent structures of this invention have a high absorbent capacity, a high density, and a flexibility which is at least equal to that of conventional fibrous webs. For these reasons, these absorbent structures are particulary suitable for use in products like diapers, incontinent pads and sanitary napkins. The high absorbent capacity and the high density make it possible to design absorbent products which are thin and yet have more than sufficient absorbent capacity to avoid the embarrassment of failure. Flexibility of the structure ensures comfort for

the wearer and a good fit of the absorbent product. The high density/low volume of the products will also result in important packaging and transport cost savings for the manufacturer.

Disposable diapers comprising the absorbent structures of the present invention may be made by using conventional diaper making techniques, but replacing the wood pulp fiber web ("air-felt") core which is typically used in conventional diapers with an absorbent structure of the present invention. Thus, a disposable diaper may be comprised of (from top to bottom) a top sheet (a non-woven, hydrophobic tissue, e.g. needle punched polyester), the absorbent structure, and a waterproof, pliable back sheet (e.g. hard polyethylene, having an embossed caliper of approximately 58 µm). Optionally, the absorbent structure may be wrapped in envelope tissue (wet strength tissue paper). Disposable diapers of this type are disclosed in more detail in U.S. Patent 3,952,745, issued April 27, 1976 to Duncan; and in U.S. Patent No. 3,860,003, issued January 14, 1975 to Buell.

Since the absorbent structures of the present invention have a higher absorbent capacity than conventional wood pulp fiber webs, the wood pulp web may be replaced with an absorbent structure of the present invention of less than equal weight. The reduced weight and the higher density combined account for a reduction in bulk by a factor 3 to 12 or more (depending on the type of hydrogel, the fiber/hydrogel ratio, and the density used).

The amount of absorbent structure used in disposable diapers is conveniently expressed as the basis weight (in g/cm$^2$) of the structure. Typically, basis weights of the absorbent structures of the present invention as used in disposable diapers range from 0.01 g/cm$^2$ to 0.05 g/cm$^2$. One way in which this invention may be used is in manufacturing diapers having both increased absorption capacity and reduced bulk as compared to conventional diapers. This can be obtained by using absorbent structures having a basis weight of from 0.018 to 0.03 g/cm$^2$. Preferred are basis weights of from 0.019 to 0.021 g/cm$^2$. A different approach is to aim at an absorbent capacity substantially equivalent to that of conventional diapers, while fully exploiting the potential of bulk reduction offered by this invention. This is generally achieved by using basis weights of from 0.01 to 0.017 g/cm$^2$. Preferred are basis weights in the range from 0.014 to 0.017 g/cm$^2$. The absorbent structures used in disposable diapers preferably have a thickness of from 0.3 mm to 2 mm, more preferably from 0.5 mm to 1 mm.

Conventional disposable diapers are usually comprised of (from top to bottom) a top sheet (a non-woven, hydrophobic tissue, e.g., needle punched polyester), a wood pulp fiber absorbent core, and a waterproof, pliable back sheet (e.g., hard polyethylene having an embossed caliper of approximately 58 µm). The absorbent capacity of such diapers is substantially increased when an absorbent structure of the present invention is placed between the wood pulp fiber core and the back sheet. When used in this manner the absorbent structures preferably have a thickness of from 0.1 mm to 1 mm. The absorbent structure used as an insert can have the same size and shape as the wood pulp fiber core, or be different. In a specific embodiment the wood pulp fiber core is hour-glass shaped (i.e., the width in the center of the core is substantially less than the width at the ends), and the absorbent structure is rectangular, having a length approximately the same as the length of the wood pulp fiber core, and a width of from 1 cm to 5 cm less than the width of the wood pulp fiber core at the narrowest point of the hourglass.

Because the absorbent structures of the present invention are highly absorbent, and yet thin and flexible, they are extremely suitable for use in sanitary napkins. As is the case with disposable diapers, sanitary napkins utilizing the present absorbent structures may be derived from conventional sanitary napkins by simply replacing the absorbent core thereof (typically a web of wood pulp fibers, with an absorbent structure of the present invention. Such replacement may be on a weight-by-weight basis, which results in a reduction in volume and a gain in capacity; or the replacement may be on a less than equal weight basis, thereby sacrificing part of the gain in absorbent capacity in favor of an even greater reduction in bulk. The absorbent structures used in sanitary napkins preferably have a thickness of from 0.1 mm to 2 mm, more preferably from 0.3 mm to 1 mm.

An example of a sanitary napkin comprises a pad of the absorbent structure of the present invention; a hydrophobic topsheet; and a fluid impervious bottom sheet. The topsheet and the backsheet are placed at opposite sides of the absorbent structure. Optionally, the absorbent structure is wrapped in envelope tissue. Suitable materials for top sheets, bottom sheet and envelope tissue are well known in the art. A more detailed description of sanitary napkins and suitable materials for use therein is found in U.S. Patent No. 3,871,378, issued March 18, 1975 to Duncan et al.

## Performance Testing

### A. Partitioning Test

Samples of absorbent structures were subjected to a partitioning test, more fully described hereinbelow. This test has been designed to measure the absorption performance of absorbent structures in competition with conventional cellulose fibrous webs, both under conditions of low liquid load and high liquid loads. The absorption fluid was "synthetic urine" (a solution of 1% NaCl, in distilled water; the surface tension of the solution was adjusted to 45 dynes/cm with 0.0025% of an octylphenoxy polyethoxy ethanol surfactant (Triton X-100, from Rohm and Hass Co.). This test has been found to be predictive of the absorption capacity under typical usage conditions of absorbent structures when used as absorbent cores in diapers.

Absorbent structures were made by metering predetermined amounts of hydrogel particles into a flow

of air containing southern soft wood slash pin fibers; the mixture was air laid on a wire mesh screen and the resulting web was densified between calender rolls to the required density. The structures had a basis weight of 0.04 g/cm$^2$. On the same equipment, webs of southern soft wood slash pine fibers were made, also having a basis weight of 0.04 g/cm$^2$ and calendered to a density of 0.1 g/cm$^2$. No hydrogel particles were added to the latter webs. The latter web served as the reference in all tests. Round samples of 6 cm diameter were punched out of the sheets of absorbent material for partitioning testing.

The partitioning tests were carried out as follows. A piece of polyethylene sheet (the kind of material generally used as a back sheet in disposable diapers) was placed on a flat, nonabsorbent surface. A round sample (6 cm diameter) of the absorbent structure to be tested was placed on top of this backsheet. On top of that was placed a piece of paper tissue of the type generally used as envelope tissue in disposable diapers. On top of the envelope tissue was placed a sample of the reference material (southern soft wood slash pine fibrous web, 0.1 g/cm$^2$ density). The top sample was wetted with a predetermined amount (about 1 g) of synthetic urine, covered with another piece of backsheet, upon which a weight of 4.4 pounds (2 kg) was placed. This weight exerts a confining pressure of 1 psi (70 × 10$^3$ N/m$^2$). After five minutes equilibration time, the weight was removed and the two samples of absorbent material were weighed separately. The "loading", defined as the amount of synthetic urine (in grams) absorbed per gram of absorbent material was calculated for each sample. The sample was then dosed with an additional dose of synthetic urine, placed back under the confining weight, equilibrated, and weighed. This was repeated several times (typically on the order of 8—10 times) so that the realtive absorption performance of the test material over a wide range of total loadings was obtained. The loading of the bottom test layer was then plotted as a function of the loading in the reference top layer.

Of particular interest are the loadings of the test layer at the points where the loading of the reference is 2.0 g/g and 4.5 g/g respectively. The loading of the test layer at the reference loading of 4.5 g/g has been found to be predictive of the loading at failure in normal use when the test material is used as a core in a disposable diaper. The loading of the test layer at a loading of the reference layer of 2.0 g/g is representative of the loading of the diaper under typical usage conditions. All experimental results reported herein are average results of duplicate or triplicate experiments.

B. Absorption/Desorption Test

The absorption properties of absorbent structures were determined by their "synthetic urine" absorption and desorption behavior. The basic procedure and the design of the apparatus are described by Burgeni and Kapur, "Capillary Sorption Equilibria in Fiber Masses", *Textile Research Journal,, 37* (1967) 362. The test is particularly useful for determining absorption kinetics.

The absorption apparatus consisted of a horizontal capillary tube, approximately 120 cm long, connected by a valve to a fluid reservoir. The end of the tube was connected by tygon tubing to a glass funnel containing an ASTM 4—8 micron frit on which the absorbent web sample was placed. The glass frit funnel was mounted on a vertical pole. The height of the frit above the capillary tube determined the hydrostatic suction being exerted on the sample. In a typical absorption/desorption experiment the volume of absorbed synthetić urine was determined as a function of hydrostatic suction, starting at 100 cm (corresponding with a hydrostatic pressure of −100 cm).

A simplifed test was developed to determine the useful capacity of an absorbent web. In this test, the absorbed volume at −25 cm hydrostatic pressure was measured ("25 cm, absorption"). Next, the frit containing the sample was lowered to zero hydrostatic pressure and the equilibrium value of sorbed volume measured ("0 cm, void volume"). Then the frit was raised again to the 25 cm mark and the absorbed volume at −25 cm in the desorption mode was determined ("25 cm, desorption").

C. Gurley Stiffness Test

The stiffness of absorbent structures was determined using a Gurley Stiffness Tester (manufactured by W. and L. E. Gurley of Troy, New York). The use of this tester is disclosed in U.S. Patent 4,354,901, issued October 19, 1982 to Kopolow.

In essence, this instrument measures the externally applied force required to produce a given deflection of a strip of material of specific dimensions, fixed at one end and having a load applied to the other end. The results were obtained as "Gurly Stiffness" values in units of grams. Each strip of absorbent material was 3.5 inches by one inch (8.9 cm × 2.5 cm).

The absorbent structures of the present invention have a Gurley Stiffness value of less than 2 g, preferably less than 1 g, when meaured on a strip having a basis weight of 0.03 g/cm$^2$.

Example I

In order to test the effect of fiber:hydrogel ratios on the partitioning performance of absorbent structures, the following absorbent structures were prepared.

Southern soft wood slash pine fibers were dry mixed with an acrylic acid grafted starch hydrogel having a weight average particle size of 250 micrometers ("Sanwet IM 1000", from Sanyo Co., Ltd., Japan) in fiber:hydrogel ratios of 100:0 (no hydrogel), 95:5, 90:10, 85:15, and 80:20. Webs having dimensions of 41 × 30 cm, and having a basis weight of 390 g/cm$^2$, were prepared in a batch type air laying equipment. The webs were compressed to a dry density of 0.3 g/cm$^2$, using a flat hydraulic press, corresponding to a

thickness of 1.3 mm.

Samples of these webs were subjected to the above-described partitioning test. The following results were obtained:

Table I

Partitioning performance of absorbent structures as a function of fiber:hydrogel ratio.

| Fiber:Hydrogel Ratio | Loading (g/g) at Reference = 2.0 g/g | Loading (g/g) at Reference = 4.5 g/g |
|---|---|---|
| 100:0 | 2.0 | 3.6 |
| 95:5 | 2.4 | 4.5 |
| 90:10 | 3.4 | 5.9 |
| 85:15 | 3.7 | 6.5 |
| 80:20 | 4.0 | 7.2 |

Table II

Absorption/desorption data[1)] as a function of fiber:hydrogel ratio

| Fiber:Hydrogel Ratio | 25 cm Absorption | 0 cm Void | 25 cm Desorption |
|---|---|---|---|
| 100:0 | 2.5 | 3.0 | 2.9 |
| 95:5 | 2.9 | 3.8 | 3.5 |
| 90:10 | 3.8 | 4.9 | 4.5 |
| 85:15 | 4.3 | 5.9 | 5.3 |
| 80:20 | 4.8 | 6.2 | 5.8 |

1) in ml/g, after 10 min. equilibration time

The data demonstate the dramatic increase in absorption capacities over a wide range of conditions which is obtained by the absorbent structures of the present invention, as compared to all-fiber structures of the same density.

Example II

For comparison, absorbent structures were prepared, using the wet-laying process described in U.S. Patent No. 4,354,901 (issued October 19, 1982 to Kopolow) as follows:

A mixture of southern slash pine wood pulp fibers and an acrylic acid grafted starch hydrogel material (Sanwet IM 1000, from Sanyo Co., Ltd., Japan) (fiber:hydrogel ratio = 80:20) was slurried in water at a consistency of 0.7%. A web was formed by straining the slurry on a wire mesh screen. The amount of slurry was such as to result in a basis weight of 0.034 g/cm$^2$. The web was dried in an oven at 100°C. The density of the dried web was 0.2 g/cm$^3$. The web was then compressed in a hydraulic press to a density of 0.38 g/cm$^3$. The resulting structure was stiff and board like.

The absorption performance of this sample was determined with the above-described partitioning test. The results are compared with those obtained with an air-laid structure prepared according to the process of the present invention. (Table III)

Table III

Partitioning performance of absorbent structures as affected by the process of making.

| Fiber:Hydrogel Ratio [1] | Process | Loading (g/g) at Ref. = 2.0 g/g | Loading (g/g) at Ref. = 4.5 g/g |
|---|---|---|---|
| 80:20 | Air-laying [2] | 4.0 | 7.2 |
| 80:20 | Wet-laying [3] | 3.4 | 4.5 |

1) density of both structures was 0.3 $g/cm^2$

2) according to the process of the present invention

3) process as described in U.S. Patent 4,354,901

The data demonstrate that the process of the present invention results in absorbent structures having absorbent properties which are far superior to those made by a wet-laying process.

Example III

The following structures were prepared using the above-described air-laying technique: an all-fiber (southern slash pine) web, density 0.1 $g/cm^3$ (sample A); an all-fiber (southern slash pine) web, density 0.3 $g/cm^3$ (sample B); a fiber (southern slash pine)/hydrogel structure (fiber:hydrogel ratio = 80:20), density 0.3 $g/cm^3$ (sample C). The hydrogel was the same as used in Examples I and II. All structures were soft and flexible.

The partitioning performance of these samples was determined using the above described partitioning test, expect that equilibration times were one minute.

Table IV

Partitioning Performance of Various Absorbent Structures

| Sample # | Loading (g/g) at Reference = 2.0 g/g | Loading (g/g) at Reference = 4.5 g/g |
|---|---|---|
| A | 1.1 | 4.4 |
| B | 2.1 | 3.9 |
| C* | 3.4 | 7.1 |

* structure according to the present invention

The partitioning data illustrates that densifying of all-fiber structure (A—B) results in a higher partitioning capacity at low loading (due to better wicking), but a lower capacity at high laoding (due to reduced void volume). An 80:20 fiber:hydrogel mixture at high density (0.3 $g/cm^3$, sample C) possesses vastly superior partitioning properties, both at low and at high loadings.

Example IV

Absorbent structures containing different types of hydrogel were made by in-line metering of dry hydrogel particles into a flow of southern softwood slash pine fibers. All hydrogel samples had a weight average particle size in the range of from 100 micrometers to 1 mm. The mixtures were formed into sheets, basis weight of 0.035 $g/cm^2$, on a wire screen. The sheets were compressed to a dry density of 0.3 $g/cm^3$.

The partitioning performance of each sheet was tested with the above-described partitioning test. The results are collected in Table V.

Table V

| Type of Hydrogel | Fiber:Hydrogel Ratio | Loading at Ref.=2.0 g/g | Loading at Ref.=4.5 g/g |
|---|---|---|---|
| None (control) | 100:0 | 2.05 | 3.60 |
| Starch, acrylonitrile[1)] | 81.2:18.8 | 3.45 | 5.35 |
| Starch, acrylonitrile[2)] | 84.6:15.4 | 2.30 | 5.40 |
| Polyacrylate[3)] | 75.0:25.0 | 5.75 | 8.65 |
| Polyacrylate[3)] | 80.8:19.2 | 5.10 | 8.10 |
| Starch, acrylonitrile[4)] | 82.7:17.3 | 4.25 | 6.10 |
| Starch, acrylonitrile[4)] | 78.7:21.3 | 4.25 | 6.10 |
| Starch, acrylonitrile[5)] | 82.6:17.4 | 4.00 | 5.40 |
| Cellulose, carboxyl[6)] | 86.0:14.0 | 2.95 | 5.14 |
| Cellulose, carboxyl[6)] | 77.9:22.1 | 3.20 | 5.40 |
| Starch, carboxyl[7)] | 82.1:17.9 | 2.20 | 4.40 |
| Starch, acrylic acid[8)] | 80.1:19.9 | 3.55 | 7.00 |
| Starch, acrylic acid[8)] | 77.7:22.3 | 4.40 | 7.40 |
| Isobutylene/ maleic anhydride copolymer[9)] | 77.6:22.4 | 4.25 | 7.75 |
| Isobutylene/ maleic anhydride copolymer[9)] | 80.0:20.0 | 4.25 | 7.45 |

1) A-100, from Grain Processing
2) A-200, from Grain Processing
3) J-550, from Grain Processing
4) SGP 147, from Henkel, U.S.A.
5) SGP 502SB, from Henkel, U.S.A.
6) Akucell 3019, from Enka, Germany
7) Foxorb 15, from Avebe, France
8) Sanwet IM 1000, from Sanyo, Japan
9) KI Gel 201, from Kuraray, Japan

As the results indicate, the presence of hydrogel particles in a densified hydrophilic fibrous web results in a significant increase in partitioning capacity, both at low load and at high load conditions.

Similar structures are prepared. wherein the southern softwood Kraft pulp fibers are replaced with hardwood Kraft pulp fibers; chemo-thermo mechanical softwood fibers; eucalyptus Kraft pulp fibers; cotton fibers; and polyester fibers. Substantially similar results are obtained.

Example V

Absorbent structures were made by the batch-type process described in Example I. Southern softwood Kraft pulp fibers were used in admixture with an acrylic acid grafted starch hydrogel ("Sanwet IM 1000",

from Sanyo Co., Ltd., Japan). This type of hydrogel has a saturation capacity for "synthetic urine" of 25X.

Samples of various fiber/hydrogel ratios were prepared. The kinetics of synthetic urine absorption of the samples was studied in the absorption/desorption apparatus described herein-above. The synthetic urine used in this test was a solution of 1% NaCl, 0.06% $MgCl_2{\cdot}6H_2O$ and 0.03% $CaCl_2{\cdot}2H_2O$ in distilled water; the surface tension of the solution was adjusted to 45 dynes/cm with 0.0025% of an octyl phenoxy polyethoxy ethanol surfactant (Triton X-100, from Rohm and Haas Co.). All absorbent structures had a density of 0.3 g/cm$^3$ and a basis weight of 0.04 g/cm$^2$. All absorption kinetics were measured under a confining pressure of 1 psi (70 × $10^3$N/m$^2$), which closely approaches real-life conditions for use in diapers.

Table VI

Absorption kinetics; hydrostatic pressure -25 cm; absorption mode

| Time (min.) | Absorption (ml/g) Fiber/hydrogel ratio (g/g) | | | | |
|---|---|---|---|---|---|
| | 100:0 | 88:12 | 73:27 | 48:52 | 34:66 |
| 5 | 2.8 | 3.8 | 4.9 | 3.8 | 2.7 |
| 10 | 2.8 | 4.2 | 5.8 | 4.6 | 3.2 |
| 30 | 2.8 | 4.4 | 6.4 | 5.9 | 4.5 |
| 60 | - | 4.5 | 6.6 | 7.0 | 5.7 |
| 360 | - | 4.6 | 7.0 | 9.8 | 9.1 |
| 720 | - | - | 7.2 | 11.0 | 10.6 |

The data indicated that the equilibrium absorption capacity increases with increasing amounts of hydrogel. The data also demonstrate, however, that the rate at which the equilibrium absorption capacity is approached becomes progressively slower with increasing amounts of hydrogel.

The optimum fiber/hydrogel ratio for this specific fiber-hydrogel system under these testing conditions appears to be around 75:25.

A similar picture is obtained with 0 cm-void volume absorption kinetics, but there are interesting differences (Table VII). Since under these test conditions the wicking properties are less important, the relative performance of the absorbent structures is to a large extent determined by the equilibrium absorption capacities of these structures. Still, a structure which has very poor absorption kinetics (i.e., fiber/hydrogel ratio of 40:60) is deficient at times 60 min. as compared to 61:39 and 53:47 fiber/hydrogel samples even under 0 cm hydrostatic pressure conditions.

Table VII

Absorption kinetics; hydrostatic pressure 0 cm

| Time (min.) | Absorption (ml/g) Fiber/Hydrogel Ratio (g/g) | | | | | |
|---|---|---|---|---|---|---|
| | 100:0 | 88:12 | 78:22 | 61:39 | 53:47 | 40:60 |
| 5 | 4.2 | 5.9 | 6.8 | 7.7 | 7.5 | 6.6 |
| 10 | 4.2 | 6.3 | 7.5 | 8.8 | 8.6 | 7.6 |
| 30 | 4.2 | 6.5 | 8.3 | 10.2 | 10.0 | 9.3 |
| 60 | - | 6.6 | 8.5 | 10.7 | 10.7 | 10.5 |
| 360 | - | 6.8 | 8.9 | 11.7 | 12.1 | 13.8 |

It is expected that, when similar samples are prepared with southern softwood Kraft pulp fibers and a hydrogel which has a saturation capacity of "synthetic urine" of about 10X, the absorption capacities will be lower for each fiber/hydrogel ratio than those given in Table VII. However, for these mixtures, a fiber/hydrogel ratio of 40:60 is expected to perform better than a fiber/hydrogel ratio of 50:50 at 5 and 10 min.

equilibration times, contrary to the picture obtained with the above hydrogel having a saturation capacity of 25X.

Example VI

Absorbent structures were made according to the process of the present invention, as described in Example I. The fiber/hydrogel weight ratio was 80:20. The Gurley Stiffness values of these structures were determined. For comparison, the Gurley Stiffness values of structures made according to the wet-laying process described in U.S. Patent No. 4,354,901 (see Example II) were determined before and after densification. (Table VIII)

Table VIII

| Sample | Density ($g/cm^3$) | Basis Weight ($g/cm^2$) | Gurley Stiffness (g) |
|---|---|---|---|
| Wet-laid | 0.1 | 0.037 | 24.4 |
| Wet-laid | 0.1 | 0.037 | 27.2 |
| Wet-laid | 0.3 | 0.033 | 5.4 |
| Wet-laid | 0.3 | 0.033 | 3.8 |
| Air-laid | 0.3 | 0.032 | 0.24 |
| Air-laid | 0.3 | 0.032 | 0.25 |
| Air-laid | 0.3 | 0.035 | 0.64 |
| Air-laid | 0.3 | 0.035 | 0.56 |

The data confirm that the Gürley Stiffness value of a wet-laid structure, which is initially very high, may be reduced by compressing the structure to a higher density, as is disclosed in U.S. Patent No 4,354,901. The data further show that the Gurley Stiffness values of the air-laid structures of the present invention are an order of magnitude lower than those of compressed wet-laid structures, and up to 2 orders of magnitude lower than those of uncompressed wet-laid structures.

Example VII

A disposable diaper utilizing an absorbent structure according to this invention was prepared as follows:

An absorbent structure prepared as in Example I was calendared to a caliper of about 0.1 cm and a density of 0.3 $g/cm^3$ as measured under a confining pressure of 0.1 PSI ($7 \times 10^3 N/m^2$). The web was cut into pads of 12 in. × 16 in. (30 × 40 cm). The pads were enveloped in wet strength tissue paper having a basis weight of 12 pounds per 3,000 square feet (20 $g/cm^2$), a dry tensile strength of 700 g/inch (275.6 g/cm) in the machine direction and 300 g/inch (118.1 g/cm) in the cross machine direction.

The eveloped pad was glued onto a 13 in. × 17 in. (33 cm × 43 cm) backsheet of embossed polyethylene film having a melt index of 3 and a density of 0.92 $g/cm^3$. The ends of the backsheet were folded over the enveloped pad and attached with glue. Finally, the absorbent pad was covered with a topsheet of a hydrophobic but water and urine pervious material. (Webline No. F 6211 from the Kendall Co. of Walpole, Massachusetts, comprised of a non-woven rayon bonded with an acrylic latex).

The diapers had superior water and synthetic urine absorption, wicking and containment characteristics.

Example VIII

Sanitary napkins employing an absorbent structure pursuant to this invention are prepared as follows:

An absorbent structure, prepared as in Example 1, is calendered to a caliper of 0.07 cm and a density of 0.4 $g/cm^3$ as measured under a confining pressure of 0.1 PSI ($7 \times 10^3 N/m^2$). The web is cut into a pad of 8 in. × 2 in. (20 cm× 3 cm) with tapered ends. On top of this pad is placed a second pad (rectangular) of 5 in. × 2 in. (13 cm × 5 cm). The combined pad structure is placed against a 8 in. × 2 in. (20 cm × 5 cm) tapered waterproof backing sheet of embossed hard polyethylene having an embossed caliper of 0.058 mm. The structure is covered with a top sheet of non-woven, needle punched polyester fabric of 0.33 Tex having a density of 0.03 $g/cm^3$ and a caliper of 2.3 mm. The thus covered structure is placed on a 9 in. × 3 in. (23 cm × 7.5 cm) bottom sheet of hydrophobic, spinbonded non-woven polyester having a measured weight of 15 $g/cm^2$. The bottom sheet is prefolded upwardly by means of heat and pressure

which bonds the superposed sheet together. The resulting absorbent structure is useful as a sanitary napkin and has superior properties of absorption and containment of menses exudate.

Example IX

Diapers containing the absorbent structures of the present invention were made as described in Example VII. Control diapers of the same design were made, using wood pulp fiber webs of 0.1 g/cm$^3$ density instead of the absorbent structures of 0.3 g/cm$^3$ density.

The diapers were worn by normal infants. The infants were allowed to play in a nursery school setting during the test. The diapers were left on the infants until leakage occurred. In order to speed up the test, the diapers were pre-loaded with a predetermined amount of synthetic urine.

After leakage occurred, the diapers were taken off and weighed to determine the amount of absorbed fluid. The loading X, defined as the amount of fluid (in grams) absorbed at the point that failure occurred per gram of absorbent material, was calculated. The results are presented in Table IX.

The absorbent core of conventional diapers (samples A, G, and I) contain about 5 times their own weight of fluid at the point of leakage. The absorbent structures of the present invention contain from 8.0 to 12.7 times their own weight of fluid at the point where leakage occurs. The data further show that the present invention makes is possible to reduce the volume of a diaper core by a factor 7 (as compared to conventional airfelt diaper cores) while maintaining the absorption capacity of the diaper (compare sample J with samples A, G and I).

Table IX

| SAMPLE | A | B | C | D | E | F | G | H | I | J |
|---|---|---|---|---|---|---|---|---|---|---|
| Absorbent Core (g) | (35.6) | (25.0) | (25.0) | (25.0) | (18.0) | (21.0) | (35.6) | (18.0) | (35.6) | (15.0) |
| Fiber (g) | 34.9 | 19.7 | 20.6 | 19.8 | 14.8 | 17.7 | 35.6 | 15.3 | 35.6 | 12.3 |
| Hydrogel (g) | --- | 4.9 | 4.5 | 4.3 | 3.3 | 3.1 | --- | 3.3 | --- | 2.7 |
| Tissue (g) | 6.0 | 6.0 | 6.0 | 6.0 | 6.0 | 6.0 | 6.0 | 6.0 | 6.0 | 6.0 |
| Total absorbent mat. | 40.9 | 30.6 | 31.1 | 30.1 | 24.1 | 26.8 | 41.6 | 24.6 | 41.6 | 21.0 |
| Fiber/hydrogel ratio | --- | 80/20 | 82/18 | 82/18 | 82/18 | 85/15 | --- | 82/18 | --- | 82/18 |
| Grams of fluid to grade 3 leakage | 194 | 238 | 263 | 208 | 245 | 244 | 179 | 230 | 181 | 183 |
| Total Abs. X to leak | 4.7 | 7.8 | 8.4 | 6.9 | 10.2 | 9.1 | 4.3 | 9.4 | 4.4 | 8.7 |
| Core (less tissue) X to failure (g/g) | 5.1 | 9.0 | 9.9 | 8.0 | 12.7 | 11.0 | 4.6 | 11.6 | 4.7 | 11.2 |
| Core thickness (mm) | 2.9 | 0.7 | 0.7 | 0.7 | 0.5 | 0.6 | 2.9 | 0.5 | 2.9 | 0.4 |
| Core basis weight ($mg/cm^2$) | 29 | 20 | 20 | 20 | 15 | 17 | 29 | 15 | 29 | 12 |

Alternatively, one may reduce the bulk of the diaper core by less than a factor 7, (e.g. by a factor 4, samples B, C and D; by a factor 5, sample F; or by a factor 6, samples E and H) and yet achieve a substantial gain in absorbent capacity as compared to conventional disposable diapers.

Example X

A diaper is prepared as described in U.S. Patent 3,860,003, Buell, issued January 14, 1975, except that, in addition to the absorbent body disclosed therein (e.g., made from air-laid wood pulp) there is inserted between said absorbent body and the backsheet an hourglass-shaped absorbent structure of the present invention. The absorbent structure is made as described in Example I. The basis weight is 0.035 g/cm$^2$; the density is 0.3 g/cm$^3$, resulting in a thickness of 1.17 mm.

Example XI

Diapers were prepared as described in U.S. Patent 3,860,003, Buell, issued January 14, 1975.

The hour-glass-shaped softwood pulp cores had the following dimensions: legnth: 15.5 in. (40 cm), width at the ears: 10.5 in. (27 cm), and width in the centre: 3.75 in. (9.5 cm).

Absorbent structures of the present invention were made with softwood fibers and acrylic acid grafted starch hydrogel having a weight average particle size of 25 micrometers ("Sanwet IM 1000", from Sanyo Co., Japan) in a fiber:hydrogel ratio of 85:15, using the process of Example I. The absorbent structures had a basis weight of 0.12 g/in. (0.019 g/cm$^2$) and a caliper of 0.03 in. (0.076 cm), which corresponds to a density of 0.25 g/cm$^3$. The structures were covered with a sheet of envelope tissue, and cut to a size of 3.5 in.$\times$ 15.5 in. (9 $\times$ 40 cm). The structures were inserted lengthwise into the above-described diapers, in between the hour-glass-shaped core and the polyethylene backing sheet, the envelope tissue against the hour-glass-shaped core.

Additional diapers were prepared by the same method, except that the dimensions of the absorbent structure insert were 2.25 $\times$ 15.5 in. (6 $\times$ 40 cm).

The inserts greatly increased the absorbent capacity for urine of the diapers.

Example XII

A soft wood fiber drylap as obtained from a conventional paper making process was sprayed with a 10% solution of a quarternary ammonium compound of the formula

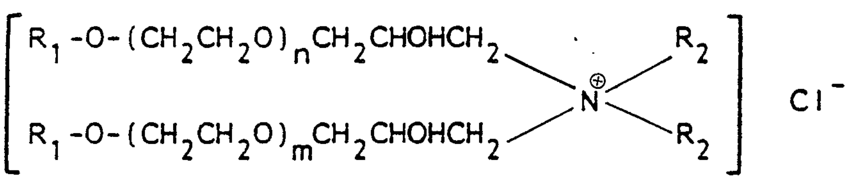

wherein n and m are integers from 2 to 10, $R_1$ is alkylaryl, and $R_2$ is alkyl having from 1 to 6 carbon atoms (Berocell 579, from Berol Chemicals, Inc., Metarie, LA).

The drylap was sprayed at a rate of 10 g solution per kg dry fiber, corresponding to 0.1% quarternary ammonium compound on the fiber. The drylap was then disintegrated, and the fibers mixed with an acrylic acid grafted starch hydrogel having a weight average particle size of 250 micrometers ("Sanwet IM 1000", from Sanyo Co., Ltd., Japan) in a fiber:hydrogel ratio of 80:20.

The fiber:hydrogel mixture was formed into an air-laid web having a basis weight of 0.13 g/in$^2$ (200 g/m$^2$) The web was calendered to a density of 0.2 g/cm$^2$, corresponding to a thickness of 0.038 in (2 mm). The absorbent structure thus obtained had excellent absorbent properties and softness. Similar structures are prepared, replacing the quaternary ammonium compound with nonionic and anionic softening agents. Structures having substantially similar properties are obtained.

The web containing the quarternary ammonium compound was cut into pads of 11 7/8 $\times$ 16 in. (30 $\times$ 41 cm). The pads were used in the manufacture of disposable diapers as described in Example VII.

**Claims**

1. An absorbent structure comprising a mixture of hydrophilic fibers and water-insoluble hydrogel in the form of discrete particles of cross-linked polymeric material in a fiber:hydrogel weight ratio of from 30:70 to 98:2, characterised in that the structure comprises a fiber-particle mixture which is air laid to form a flexible substantially unbonded structure with a moisture content of less than 10% by weight and a density of from 0.15 to 1 g/cm$^3$.

2. An absorbent structure according to claim 1 which has a Gurley Stiffness value of less than 2 g.

3. An absorbent structure according to either one of claims 1 and 2 having a fiber/hydrogel weight ratio of from 50:50 to 95:5, preferably of from 75:25 to 90:10.

4. An absorbent structure according to any one of claims 1—3 having a density of from 0.15 to 0.6 g/cm$^3$, preferably of from 0.25 to 0.4 g/cm$^3$.

5. An absorbent structure according to any one of claims 1—4 wherein the hydrophilic fibers are wood pulp fibers.

6. An absorbent structure according to any one of claims 1—5 wherein the water-insoluble hydrogel is selected from hydrolyzed acrylonitrile grafted starch, arcylic acid grafted starch, polyacrylates, copolymers of isobutylene and maleic anhydride and mixtures thereof.

7. An absorbent structure according to any one of claims 1—6 the hydrogel particles have an average particle size of from 30 microns to 4 mm, preferably of from 50 microns to 1 mm.

8. An absorbent structure according to any one of claims 1—7, further comprising from 0.01% to 0.5% by weight of the hydrophilic fibers of a quarternary ammonium compound of the formula

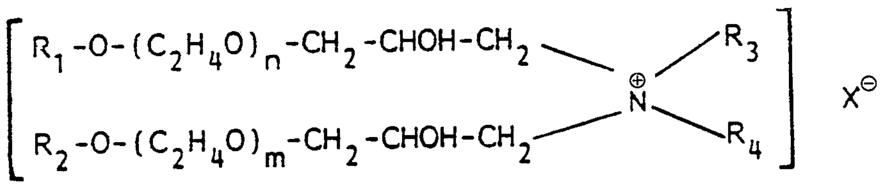

wherein $R_1$ and $R_2$ are hydrocarbyl groups containing from 8 to 22 carbon atoms, $R_3$ and $R_4$ are alkyl having from 1 to 6 carbon atoms; n and m are integers from 2 to 10, and X is halogen.

9. A process for making a flexible absorbent structure, having a composition in accordance with any one of claims 1—8 comprising the following steps:

(a) air-laying into a web a dry mixture of the hydrophilic fibers and water-insoluble hydrogel particles: and

(b) compressing the web to a density of from 0.15 to 1 g/cm$^3$.

10. A disposable absorbent produce comprising:

(a) a liquid impervious backing sheet;

(b) a hydrophobic top sheet; and

(c) an absorbent structure according to any one of claims 1—8 said structure being placed between the backing sheet and the top sheet.

11. A disposable absorbent product according to claim 10 in the form of a disposable diaper wherein the absorbent structure has a basis weight of from 0.01 to 0.05 g/cm$^2$.

12. A disposable diaper according to claim 10 wherein the absorbent structure has a thickness of from 0.3 mm to 2 mm, preferably from 0.5 mm to 1 mm.

13. A disposable diaper according to either one of claims 11 and 12, further comprising a wood pulp fiber absorbent core which is placed between the hydrophobic to sheet (b) and the absorbent structure (c).

14. A disposable diaper according to claim 13 wherein the wood pulp fiber absorbent core is hour-glass shaped and the absorbent structure (c) is rectangular.

15. A disposable diaper according to any one of claims 11—14 wherein the absorbent structure is wrapped in envelope tissue.

16. A disposable diaper according to any one of claims 11—15 wherein the absorbent structure is hour-glass-shaped.

**Patentansprüche**

1. Ein absorbierendes Gebilde, enthaldend ein Gemisch von hydrophilen Fasern und wasserunlöslichem Hydrogel in der Form von diskreten Teilchen aus vernetztem Polymermaterial in einem Gewichtsverhältnis von Faser:Hydrogel von 30:70 bis 98:2, dadurch gekennzeichnet, daß das Gebilde ein Faser-Teilchen-Gemisch enthält, das unter Bildung eines biegsamen, im wesentlichen ungebundenen Gebildes mit einem Feuchtikeitsgehalt von weniger als 10 Gew.-% und einer Dichte von 0,15 bis 1 g/cm$^3$ mit einem Luftstrom abgeschieden ist.

2. Ein absorbierendes Gebilde nach Anspruch 1, das einen Gurley-Steifheitswert von weniger als 2 g hat.

3. Ein absorbierendes Gebilde nach einem der Anspruche 1 und 2, mit einem Gewichtsverhältnis von Faser/Hydrogel von 50:50 bis 95:5, vorzugsweise von 75:25 bis 90:10.

4. Ein absorbierendes Gebilde nach einem der Ansprüche 1 bis 3, mit einer Dichte von 0,15 bis 0,6 g/cm$^3$, vorzugsweise von 0,25 bis 0,4 g/cm$^3$.

5. Ein absorbierendes Gebilde nach einem der Ansprüche 1 bis 4, wobei die hydrophilen Fasern Holzzellstoffasern sind.

6. Ein absorbierendes Gebilde nach einem der Ansprüche 1 bis 5, wobei des wasserunlösliche Hydrogel aus hydrolysierter, Acrylnitril-gepfropfter Stärke, Acrylsäure-gepfropfter Stärke, Polyacrylaten, Copolymeren von Isobutylen und Maleinsäureanhydrid, und Gemischen davon, ausgewählt ist.

7. Ein absorbierendes Gebilde nach einem der Ansprüche 1 bis 6, wobei die Hydrogelteilchen eine durchschnittliche Teilchengröße von 30 µm bis 4 mm, vorzugsweise von 50 µm bis 1 mm, haben.

8. Ein absorbierendes Gebilde nach einem der Ansprüche 1 bis 7, welches weiterhin von 0,01% bis 0,5%, bezogen auf das Gewicht der hydrophilen Fasern, einer quarternären Ammoniumverbindung der Formel

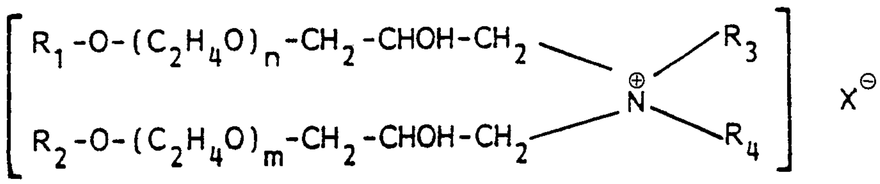

enthält, worin $R_1$ und $R_2$ 8 bis 22 Kohlenstoffatome ethaltende Kohlenwasserstoffgruppen sind, $R_3$ und $R_4$ Alkylgruppen mit 1 bis 6 Kohlenstoffatomen sind; n und m ganze Zahlen von 2 bis 10 sind, und X Halogen ist.

9. Ein Verfahren zur Herstellung eines biegsamen, absorbierenden Gebildes, mit einer Zusammensetzung gemäß einem der Ansprüche 1 bis 8, umfassend die folgenden Stufen:

(a) Ablegen eines trockenen Gemisches der hydrophilen Fasern und wasserunlöslichen Hydrogelteilchen in einem Luftstrom zu einer Faserbahn; und

(b) Zusammenpressen der Faserbahn auf eine Dichte von 0,15 bis 1 g/cm³.

10. Ein absorbierendes Wegwerfprodukt, umfassend:

(a) eine flüssigkeitsundurchlässige Unterlage;

(b) eine hydrophobe Abdecklage; und

(c) ein absorbierendes Gebilde nach einem der Ansprüche 1 bis 8, wobei das genannte Gebilde zwischen der Unterlage und der Abdecklage angeordnet ist.

11. Ein absorbierendes Wegwerfprodukt nach Anspruch 10, in der Form einer Wegwerfwindel, wobei das absorbierende Gebilde in Flächengewicht von 0,01 bis 0,05 g/cm² hat.

12. Eine Wegwerfwindel nach Anspruch 10, wobei das asorbierende Gebilde eine von 0,3 mm bis 2 mm, vorzugsweise von 0,5 mm bis 1 mm, hat.

13. Eine Wegwerfwindel nach einem der Ansprüche 11 und 12, welche weiterhin einen absorbierenden Kern aus Holzzellstoffasern enthält, welcher Kern zwischen der hyrophoben Abdecklage (b) und dem absorbierenden Gebilde (c) angeordnet ist.

14. Eine Wegwerfwindel nach Anspruch 13, wobei der absorbierende Kern aus Holzzellstoffasern sanduhrförmig und das absorbierende Gebilde (c) rechteckig ist.

15. Eine Wegwerfwindel nach einem der Ansprüche 11 bis 14, wobei das absorbierende Gebilde in einen dünnen Umhüllungsstoff eingepackt ist.

16. Eine Wegwerfwindel nach einem der Ansprüche 11 bis 15, wobei das absorbierende Gebilde sanduhrförmig ist.

**Revendications**

1. Structure absorbante comprenant un mélange de fibres hydrophiles et d'hydrogel insoluble dans l'eau sous forme de particules discrètes de substance polymère réticulée, dans un rapport pondéral fibre:hydrogel de 30:70 à 98:2, caractérisée en ce que la structure comprend un mélange fibres-particules qui est étalé par jet d'air pour former une structure souple essentiellement non liée ayant une teneur en humidité inférieure à 10% en poids et une masse volumique de 0,15 à 1 g/cm³.

2. Structure absorbante selon la revendication 1, qui possède une valeur de rigidité Gurley inférieure à 2 g.

3. Structure absorbante selon l'une ou l'autre des revendications 1 et 2, ayant un rapport pondéral fibre/hydrogel de 50:50 à 95:5, de préférence de 75:25 à 90:10.

4. Structure absorbante selon l'une quelconque des revendications 1—3, ayant une masse volumique de 0,15 à 0,6 g/cm³, de préférence de 0,25 à g/cm³.

5. Structure absorbante selon l'une quelconque des revendications 1—4, dans lequelle les fibres hydrophiles sont des fibres de pâte de bois.

6. Structure absorbante selon l'une quelconque des revendications 1—5, dans lequelle l'hydrogel insoluble dans l'eau est choisi parmi l'amidon greffé à l'acétonitrile hydrolysé, l'amidon greffé à l'acide acrylique, les polyacrylates, les copolymères d'isobutylène et d'anhydride maléique et leurs mélanges.

7. Structure absorbante selon l'une quenconque des revendications 1—6, dans laquelle les particules d'hydrogel ont une granulométrie moyenne de 30 microns à 4 mm, de préférence de 50 microns à 1 mm.

8. Structure absorbante selon l'une quelconque des revendications 1—7, comprenant en outre de 0,01% à 0,5% en poids des fibres hydrophiles d'un composé d'ammonium quaternaire de formule

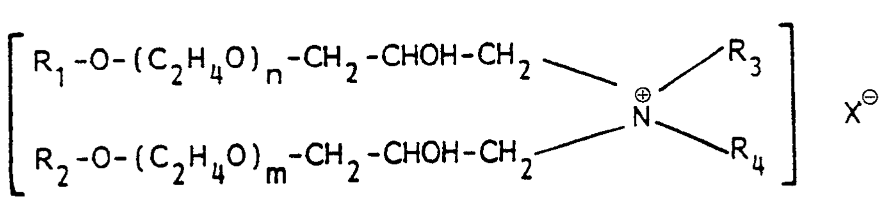

dans laquelle $R_1$ et $R_2$ sont des groupes hydrocarbonés contenant de 8 à 22 atomes de carbone, $R_3$ et $R_4$ sont des groupes alkyle comportant de 1 à 6 atomes de carbone; n et m sont des nombres entiers de 2 à 10 et X est un halogène.

9. Procédé de fabrication d'une structure absorbante flexible ayant une composition selon l'une quelconque des revendications 1—8, comprenant les étapes suivantes:

(a) formation d'une nappe par jet d'air d'un mélange sec des fibres hydrophiles et des particules d'hydrogel insoluble dans l'eau;

(b) compression de la nappe jusqu'à une masse volumique de 0,15 à 1 g/$cm^3$.

10. Produit absorbant à jeter après usage comprenant:

(a) une feuille de fond imperméable aux liquides;

(b) une feuille de dessus hydrophobe; et

(c) une structure absorbante selon l'une quelconque des revendications 1—8, cette structure étant placée entre la feuille de fond et la feuille de dessus.

11. Produit absorbant à jeter après usage selon la revendication 10 sous forme d'une couche à jeter après usage, dans lequel la structure absorbante a un grammage de 0,01 à 0,05 g/$cm^2$.

12. Couche à jeter après usage selon la revendication 10, dans laquelle la structure absorbante a une épaisseur de 0,3 mm à 2 mm, de préférence de 0,5 mm à 1 mm.

13. Couche à jeter après usage selon l'une quelconque des revendications 11 et 12, comprenant en outre une âme absorbante en fibres de pâte de bois qui est placée entre la feuille de dessus hydrophobe (b) et la structure absorbante (c).

14. Couche à jeter après usage selon la revendication 13, dans laquelle l'âme absorbante de fibres de pâte de bois a la forme d'un sablier et la structure absorbante (c) est rectangulaire.

15. Couche à jeter après usage selon l'une quelconque des revendications 11—14, dans laquelle la structure absorbante est enveloppée dans une enveloppe de tissu ouate.

16. Couche à jeter après usage selon l'une quelconque des revendications 11—15, dans laquelle la structure absorbante a la forme d'un sablier.